# EUROPEAN PATENT APPLICATION

(11) **EP 1 357 110 A1**
(43) Date of publication of application: **29.10.2003**
(21) Application number: 02009121.1
(22) Date of filing: 24.04.2002
(51) Int. Cl.: C07D 207/32, C12P 17/10, C07F 7/18, C07C 247/12

(54) **Synthesis for the preparation of porphobilinogen, the compound as such as stabilized salt and intermediates thereof**

(71) Applicant: Neier, Reinhard, 2068 Hauterive (CH)
(72) Inventor: Neier, Reinhard, 2068 Hauterive (CH); Soldermann-Pissot, Carole, 2000 Neuenburg (CH)

(57) **Abstract**

The invention relates to a new inventive process sequence of steps a) to e) as claimed in Claim 1 for the preparation of porphobilinogen and intermediates per se as important precursors such as 3-[4-methoxycarbonylmethyl-5-(protected amino-methyl)-1 H-pyrrol-3-yl]-propionic acid methyl ester of formula 7 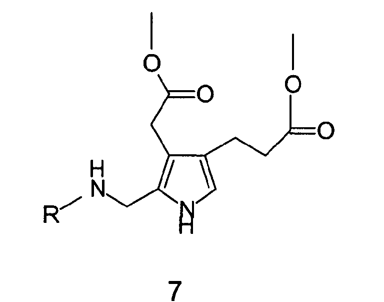 wherein R is a protective group,
and 3-[4-Carboxymethyl-5-(protected amino-methyl)-1 H-pyrrol-3-yl]-propionic acid di-alkali metal salt of formula 8 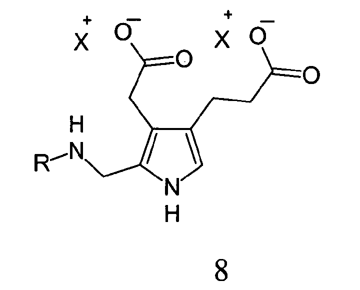 and especially 3-[4-carboxymethyl-5-(phenylacetyl-amino-methyl)- 1H-pyrrol-3-yl]-propionic acid di-lithium salt of formula 8a 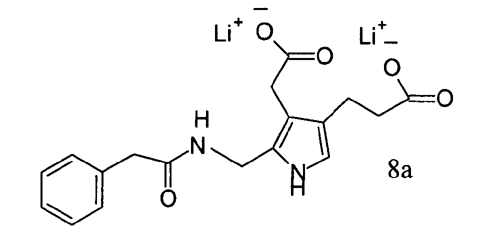 and of carboxylate 4-(2-carboxy-ethyl)-3-carboxymethyl-1H-pyrrrol-2-yl-methyl-ammonium of formula 9 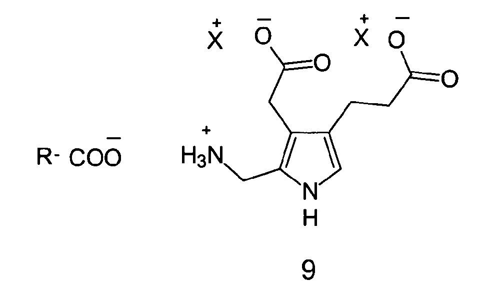 and of phenylacetate 4-(2-carboxy-ethyl)-3carboxymethyl-1 H-pyrrol-2-ylmethyl-ammonium of formula 9a 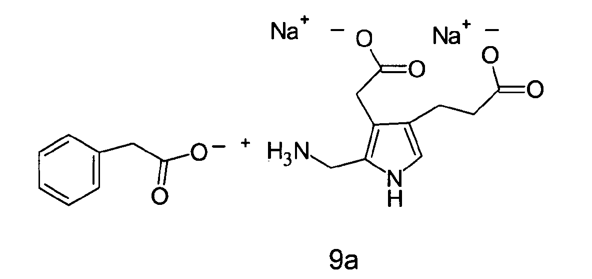 and the use thereof for the preparation of porphobilinogen.

## Description

The invention relates to a new and inventive process sequence for the preparation of porphobilinogen and an important precursor, new intermediates and to the compound as such as stabilized salt per se and the use thereof.

Porphobilinogen is a naturally occurring heterocyclic compound that is the biosynthetic precursor to nearly all biologically important tetrapyrroles, e.g. hemoglobin, chlorophyll, corrins and many other brightly coloured pigments of life.
Although porphobilinogen was initially isolated and characterized by R.G. Westall, Nature 170, 164 (1952) already some fifty years ago and the subsequent structure proof of Cookson and Rimington, Biochem. J. 57, 476 (1954) and the first practical synthesis by the Knorr method was carried out by Jackson and MacDonald, Can. J. Chem. 35, 715 (1957) some 5 years later, the interest for said compound was increasing rapidly.

Partly this is because of its utility as a building block for complex bilanes and due to its potential increasing medical value (photodynamic therapy for treatments of tumors and treatment of acute Pb poisoning). The synthesis was improved by Arsenault and MacDonald, Can. J. Chem. 39, 2043 (1961) to give an overall yield of up to 1%.

Other approaches by H. Plieninger et al., Chem. Ber. 101, 240 (1968) and to some extent Kenner et al., J. Chem. Soc. Chem. Commun. 43 (1973) increased the yield of the MacDonald key intermediate and thus the overall yield of the complete synthesis in very low percentages.
Frydman et al., J. Am. Chem. Soc. 91, 2338 (1969) developed a different route by extending a pyridone derivative by ring closure to an azaindole which was eventually converted to the porphobilinogen lactam by several steps. Major improvements to this approach were made by Battersby et al., J. Chem. Soc. Chem. Commun. 493 (1975) giving an overall yield of almost up to 15%, starting from a commercially available pyridine derivative.
H.J. Anderson et al., Can. J. Chem. 61, 2415 (1983) developed a synthetic strategy which enables pyrrole to be used as starting compound for the synthesis of 2,3,4-trisubstituted pyrroles of the type usually used to build-up porphyrins. The total synthesis of nine steps has been successfully accomplished increasing the overall yield to reach about 6%, not overwhelming due to the length of the synthesis of nine steps.
M. Adamczyk et al., Tetrahedron Lett. 36, 9121 (1995), more precisely reported in Tetrahedron Lett. 52,14689 (1996) synthesized porphobilinogen from 2-cyano-3,4-substituted pyrrole, which was obtained by condensation of an α-acetoxynitro compound with isocyanoacetonitrile. The reaction started from commercially available methyl 4-nitrobutyrate in seven steps. The yield was defined as adequate relating to the difficulties of a lengthy seven step synthesis. An improved, but very similar synthesis was developed by the very same M. Adamczyk et al. a few months later, Tetrahedron Lett. 37, 2325 (1996) starting from the 2-carboxy-3,4-substituted pyrrole, the porphobilinogen like hapten was prepared by condensation of an α-acetoxynitro compound with benzyl isocyanoacetonitrile and further functional group transformations and passing the lactam formation as in the synthesis mentioned above. As above the yield was defined as adequate relating to the length of the seven steps synthesis but did not exceed overall 6,3%.
P.A. Jacobi et al., J. Am. Chem. Soc. 123, 9307 (2001) have developed a synthesis of porphobilinogen via a novel ozonide cleavage reaction by passing porphobilinogen lactam methylester, which has been prepared in seven steps (overall yield 13-19%) beginning with furfurylamine. The key intermediate is the 7-oxonorbornene which was derived from utilizing a tandem Johnson ortho ester Claisen rearrangement followed by intramolecular Diels-Alder cyclization.
The conversion of the obtained 7-oxonorbornene was then accomplished by the above mentioned novel ozonide cleavage/oxidation reaction which resulted in tetrahydrofurans in the proper oxidation state for direct aminolysis to the corresponding lactams of porphobilinogen.

This synthesis could be defined as one of the most sophisticated synthesis avoiding the use of toxic and or expensive reagents with the disadvantage of very lengthy synthesis covering many steps and resulting only in an average, adequate yield.

Those above reported synthesis and additionally herein not discussed published synthesis are of less commercial interest, due to the commercially non-available or difficult available starting materials and to the very lengthy synthesis.

The embodiment and goal of the present invention is to overcome all the drawbacks of the known processes and to provide a more simple and improved process for the preparation of porphobilinogen and its precursors and very special stable salts, e.g. the di-lithium salt on the basis of definitely high overall yield which is feasible on an industrial scale too and provides a product having a high purity.
Furthermore the precursor obtained is stable with a long shelf-life, since it is common knowledge that porphobilinogen as free amino compound is very unstable.
According to a further aspect of the invention there is provided a conventional process for obtaining stable salts and especially as stable salt claimed per se the di-lithium salt of porphobilinogen and the use thereof.

A short synthesis for the preparation of porphobilinogen (PBG) has been developed, comprising only five steps:
a) reacting lithium hexamethyldisilazane according to following reaction scheme with a 5-hydroxy-4-oxo-pentanoic acid methyl ester of formula 1 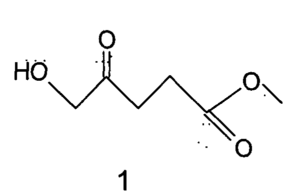 in an inert solvent under cooling at lower temperatures, at temperatures between 0° to - 100°C, in the presence of trimethylsilyl chloride and methyljodide obtaining 4,5-bis-trimethylsilanyloxy-pent-3-enoic acid methyl ester of formula 2 in cis- or trans form 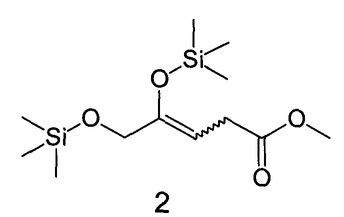 The compound of formula 2 is a new compound and is used as intermediate for the next step b) of the process sequence.
b) The obtained 4,5-bis-trimethylsilanyloxy-pent-3-enoic acid methylester of above formula 2 is reacted with 5-azido-4,4-dimethoxy-pentanoic acid methyl ester of formula 3 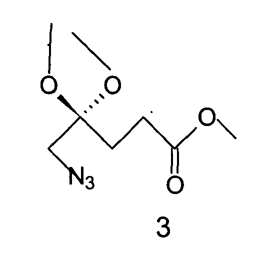 in an inert solvent, in the presence of an acid catalyst and cooling at low temperature of from 0° to -100°C, obtaining 4-azidomethyl-3-(2-hydroxy-acetyl)-4-methoxy-heptanedioic acid dimethyl ester of formula 4 in racemic and stereoisomeric form, e.g. 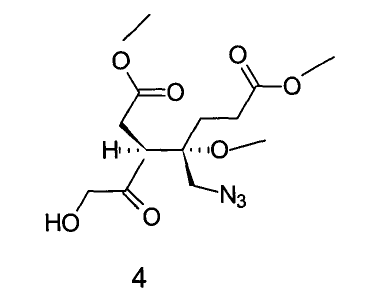 The compound of formula 4 is a new compound and is used as further intermediate for the next step c) of the process sequence.
c) 4-azidomethyl-3-(2-hydroxyacetyl)-4-methoxy-heptanedioic acid methyl ester of formula 4 in the presence of triphenylphosphin and dialkylazodicarboxylate and the like in an inert solvent is reacted with HN3 or diphenylphosphorylazide and the like in an inert solvent under cooling obtaining 3-(2-azido-acetyl)-4-azidomethyl-4-methoxyheptanedioic acid methyl ester of formula 5 in racemic and stereoisomeric form, e.g. 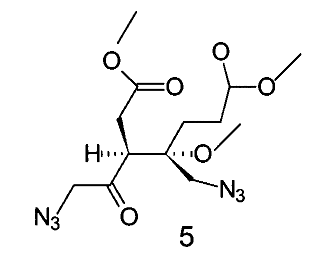 The compound of formula 5 is a new compound and is used as further intermediate for the following step d) of the process sequence.
d) The obtained 3-(2-azido-acetyl)-4-azidomethyl-4-methoxyheptanedioic acid dimethylester of formula 5 is reacted with a pre hydrogenated solution of phenylacetic acid pentafluorophenyl ester of formula 6 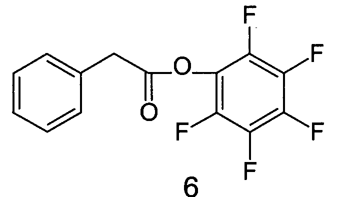 in an inert solvent to afford 3-[4-methoxycarbonylmethyl-5-(protected-amino-methyl)-1H-pyrrol-3-yl]-propionic acid methyl ester of formula 7 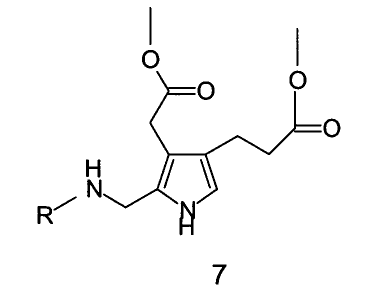 wherein R is a protective group.
   R as a protective group is, e.g. an acyl, unsubstituted or substituted acetyl, e.g. phenylacetyl, benzyloxycarbonyl, tert.-butoxycarbonyl (BOC) or fluoren-9-yl-methoxycarbonyl (Fmoc) group.
   The obtained compounds of formula 7 are new compounds and very stable precursors of porphobilinogen used as intermediate for the last step e) of the process sequence.
e) The obtained 3-[4-methoxycarbonylmethyl-5-(protected amino-methyl)-1 H-pyrrol-3-yl]-propionic acid dimethyl esters of formula 7 are converted with an aqueous/alcoholic alkaline solution into the di-alkali metal salt of formula 8 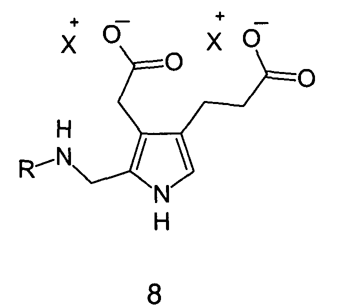
wherein R is a protective group as given above and X is an alkali metal, and thereafter is hydrolyzed under mild enzymatic conditions or is converted directly by saponification and enzymatic deprotection of a compound of formula 7 to afford porphobilinogen of formula 9 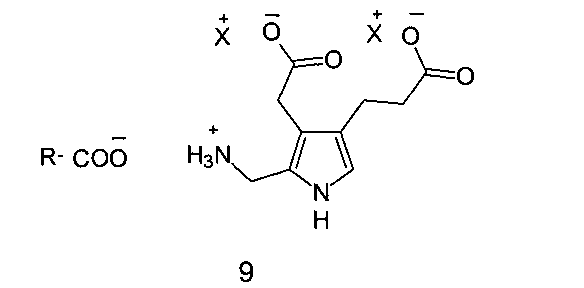 wherein R is a protective group as given above and X is an alkali metal, as carboxylate 4-(2-carboxy-ethyl)-3-carboxymethyl-1H-pyrrol-2-yl-methyl-ammonium, and further, if desired, converting the obtained salt into the free amino compound by conventional means, if desired, converting an obtained free amino compound of formula 9 to another corresponding pharmaceutical salt by conventional means.

The reaction steps a) to e) of the above mentioned process sequence for the preparation of porphobilinogen (PBG) are carried out in more detail described as follows:
a) lithium hexamethyldisilazane is reacted with 5-hydroxy-4-oxo-pentanoic acid methyl ester of formula 2 in an inert solvent, e.g., methanol, ethanol, propanol, dichloromethane, dichloroethane especially in tetrahydrofuran under cooling at lower temperatures, at temperatures between 0° and -100°C, especially at temperatures between -70° and -80°C in the presence of trimethylsilylchloride and methyl jodide.
b) 4,5-bis-trimethylsilanyloxy-pent-3-enoic acid methylester of formula 2 is reacted with 5-azido-4,4-dimethoxy-pentanoic acid methyl ester of formula 3 in an inert solvent, e.g. an alcohol, THF, DMF, dichloroethane and mixtures thereof, especially in dichloromethane in the presence of titanium chloride under cooling at low temperatures of from 0° to - 100°C, especially at temperatures between -70° and -80°C.
c) 4-azidomethyl-3-(2-hydroxyacetyl)-4-methoxy-heptanedioic acid methyl ester of formula 4 is reacted in the presence of triphenylphosphin and dialkylazodicarboxylate, especially diisopropylazodicarboxylate in an inert solvent, e.g. methanol, ethanol, propanol, tetrahydrofuran, especially in benzene with HN3 in an inert solvent mentioned before, especially in benzene under cooling at temperatures of from -20° to +20°C, especially at temperatures of from +5° to +15°C.
d) 3-(2-azido-acetyl)-4-azidomethyl-4-methoxy-heptanedioic acid dimethylester of formula 5 is reacted with a pre hydrogenated solution of phenylacetic acid pentafluorophenyl ester of formula 6 in an inert solvent, e.g. methanol, ethanol, propanol, THF or benzene, dichloromethane and dichloroethane and mixtures thereof, especially in methanol.
e) 3-[4-methoxycarbonylmethyl-5-(protected aminomethyl)-1H-pyrrol-3-yl]-propionic acid methyl ester of formula 7 is hydrolyzed in aqueous/alcoholic alkaline solution obtaining a di-alkali metal salt of formula 8 and especially in aqueous/methanolic solution in the presence LiOH.H2O, obtaining the Li-salt of formula 8a (see below)
and thereafter hydrolyzing with an enzyme, especially penicillin G acylase or by treating a compound of formula 7 by enzymatic saponification (hydrolysation) obtaining porphobilinogen which is isolated as carboxylate 4-(2-carboxy-ethyl)-3-carboxymethyl-1H-pyrrol-2-yl-methyl-ammonium of formula 9 or when R is a phenylacetyl group as phenylacetyl 4-(2-carboxy-ethyl)-3-carboxymethyl-1H-pyrrol-2-yl-methyl-ammonium of formula 9a

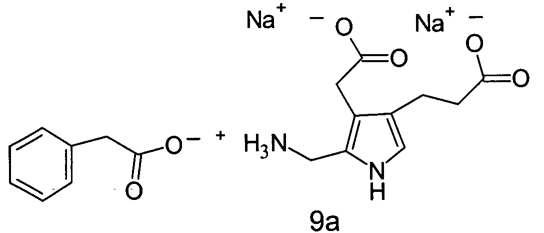

As already mentioned above the precursors of formula 7 or the di-alkali metal salts of porphobilinogen of formula 8 and especially of the above mentioned di-lithium salt of formula 8a 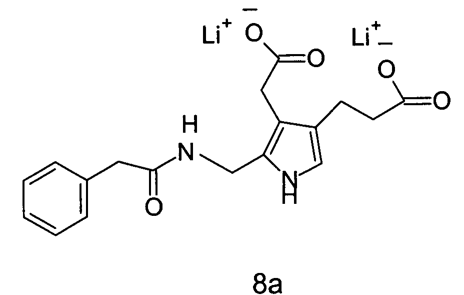 are as such new stable compounds which need not to be stored as usually under low temperatures.

The process sequence comprising five steps for the short synthesis of porphobilinogen (PBG) is working surprisingly under extremely mild conditions avoiding all harsh conditions and is a new and improved, economical and commercially feasible method of preparing porphobilinogen of formula 9 and 9a.

The phenylacetyl protected porphobilinogen of formula 7 is obtained as crystalline compound and is as such as reported before very stable. The deprotected pyrrole is a "ready to use" porphobilinogen precursor which can be deprotected to give unexpected high yields of PBG under very mild conditions. The yield of every single step a) to e) exceeds easily 70% and in some cases reaches 85% and thus a high overall yield is surprisingly obtained in a short and chemically simple five steps synthesis.
Furthermore porphobilinogen is thus obtained in high purity, no further purification is necessary and may be used immediately for treatment.

A second aspect of the present invention are the novel intermediates of formula 2, 4, 5, and 7, which are prepared according to the reactions described under steps a) to e) and are useful for the preparation of porphobilinogen.

Porphobilinogen for medical treatment may be administered by every known route and may be selected from the group consisting of the intravenous route, the intraarterial route, the intracutaneus route, the subcutaneous route, the oral route, the buccal route, the intramuscular route, the anal route, the transdermal route, the intradermal route, the intratechal route and the like.

Also sustained release formulations may be performed involving biodegradable microspheres, such as microspheres comprising polyacrylic acid, polyglycolic acid or mixtures of these.

The following non limiting examples illustrate the inventor's preferred method for preparing the claimed compounds of the invention, which should not be construed as limiting the scope of this invention.

### Example 1:

### 4,5-Bis-trimethylsilanyloxy-pent-3-enoic acid methyl ester (2)

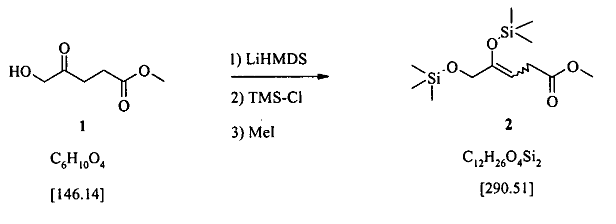

To a mechanically stirred solution of 66 ml (66 mmol) of lithium hexamethyldisilazane (LiHMDS), 1N in tetrahydrofuran (THF) cooled to -78 °C was added via cannula a solution of 4.384 g (30 mmol) of 5-Hydroxy-4-oxo-pentanoic acid methyl ester (**1**) in 70 ml of dry THF. The resulting red orange solution was stirred for 2 h and then quenched with 7.17 g (66 mmol) of trimethylsilyl chlorid (TMS-CI) and 4.117 g (66 mmol) of methyl iodide (Mel) was then added after 1 h. The cooling bath was then removed and the yellow solution was allowed to warm to room temperature (R.T.). The organic mixture was concentrated under reduced pressure and 200 ml of pentane was added. The resulting salts were removed by filtration over a celite pad. After evaporation to dryness the yellow residue was purified by bulb to bulb distillation under reduced pressure to afford 6.1 g (70 %) of 4,5-Bis-trimethylsilanyloxy-pent-3-enoic acid methyl ester (**2**). 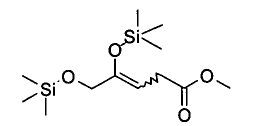 **R**_{**f**} (n-hexan /EtOAc 1:1): 0.74 (KMnO₄)
**IR (neat) :** 2957m, 2902m, 1744s, 1677m, 1630w, 1436m, 1364m, 1317m, 1252s, 1196m, 1167m, 1118m, 1064m, 1014m, 948w, 846vs, 756m, 692w, 647w, 494vw, 410w
^{**1**}**H-NMR (400 MHz, CDCl**_{**3**}**, 298 K)** : 4.96 (tt, 1H, ³J= 7.1, ⁴J=1.0, HC(3)), 3.96 (d, 2H, ⁴J=1.0, H₂C(5)), 3.68 (s, 1H, H₃C(1¹)), 3.08 (d, 2H, ³J= 7.1, H₂C(2)), 0.21 (s, 9H, Si(CH₃)₃), 0.14 (s, 9H, Si(CH₃)₃)
^{**13**}**C-NMR (100 MHz, CDCl**_{**3**}**, 298 K)** : 172.63 (C(1)), 151.45 (C(4)), 100.37 (C(3)), 63.80 (C(5)), 51.62 (C(1¹)), 30.66 (C(2)), 0.64 (Si(**C**H₃)₃), -0.58 (Si(**C**H₃)₃)
**MS** (ESI(+)) : [M+Na]⁺ = 313.3
**HR-MS:** 313.1263670 ([M+Na]⁺ ; calc. 313.1261835)

### Example 2

### 4-Azidomethyl-3-(2-hydroxy-acetyl)-4-methoxy-heptanedioic-acid dimethyl ester (4):

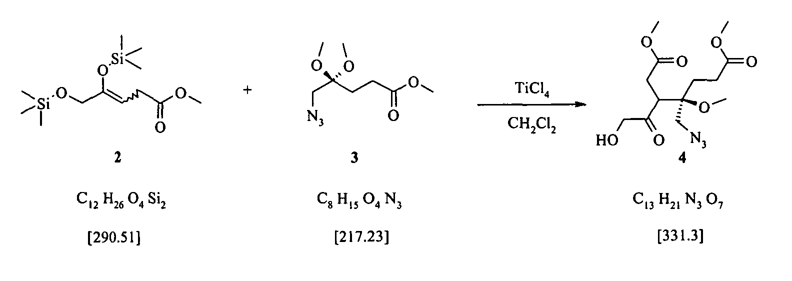

In a 200 ml three-necked flask fitted with two dropping funnels, a solution of 5.497 g (18.92 mmol) of 4,5-Bis-trimethylsilanyloxy-pent-3-enoic acid methyl ester (**2**) in 35 ml dichloromethane (CH₂Cl₂) was cooled to - 78°C. A solution of 4.932 g (22.7 mmol) of 5-Azido-4,4-dimethoxy-pentanoic acid methyl ester (**3**) in 35 ml CH₂Cl₂ was slowly added and the resulting mixture was treated with vigorous stirring with a solution of 10.4 ml (94.6 mmol) of titanium tetrachloride (TiCl₄) in 35 ml de CH₂Cl₂. The red brown solution was allowed to warm slowly to - 55°C and was then stirred for 12 h.
The reaction mixture was poured into 125 ml 2N NaOH diluted with 125 ml chloroform (CHCl₃). The aqueous layer was extracted 2 x 125 ml CHCl₃ and the combined organic extracts were then washed with 375 ml a saturated NH₄Cl solution, dried over Na₂SO₄ and concentrated to dryness under reduced pressure. The residue was chromatographed (silica gel; n-hexane / EtOAc, 1:1) to afford 4.387 g (70%) of 4-Azidomethyl-3-(2-hydroxy-acetyl)-4-methoxy-heptanedioic acid dimethyl ester (**4**) as a yellow oil. 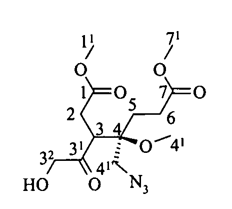 **R**_{**f**} **:** 0.25 (n-hexan /EtOAc 1:1)
**IR (neat):** 3491m, 2955s, 2841m, 2108vs, 1732vs, 1438s, 1278s, 1202s, 1179s, 1001s, 1020s, 891m, 776w, 733w, 649w, 555w, 408w ^{**1**}**H-NMR (400 MHz, CDCl**_{**3**}**, 298 K):** 4.47 et 4.34 (2xd AB system, ²J= 19.1, 1H each, Hₐ resp. H_{b}C(3²)), 3.68 (s, 3H, H₃C(7¹)), 3.64 (s, 3H, H₃C(1¹)), 3.52 et 3.35 (2xd AB system, ²J= 13.3, 1H each, Hₐ resp. H_{b}C(4^{1'})), 3.31 (dd, ³J(3, H_{b}-C(2))= 2.5, ³J(3, H_{b}-C(2))= 12.0, 1H, HC(3)), 3.24 (s, 3H, OC(4¹)H₃), 3.09 (s broad, 1H, OH), 2.97 (dd, ³J(HₐC(2), 3)= 12.0, ²J(HₐC(2), H_{b}C(2))= 17.3, 1H, HₐC(2)), 2.45 (dd, ³J(H_{b}-C(2), 3)= 2.5, ²J(H_{b}C(2), HₐC(2))= 17.3, 1H, HbC(2)), 2.40 (ddd, ²J(HₐC(6), H_{b}C(6))≈16, ³J(Hₐ-C(6), H_{b}-C(5))=9.9, ³J(Hₐ-C(6), Hₐ-C(5))=6.2, 1H , HₐC(6)), 2.33 (ddd, ²J(H_{b}C(6), HₐC(6))≈15.9, ³J(H_{b}-C(6), Hₐ-C(5))=9.4, ³J(H_{b}-C(6), Hₐ-C(5))=6.2, 1H, HbC(6)), 2.12 (ddd, ²J(HₐC(5), H_{b}C(5))≈15.4, ³J(HₐC(5), H_{b}C(6))=9.4, ³J(HₐC(5), HₐC(6))=6.1, 1H, HₐC(5)), 1.81 (ddd, ²J(H_{b}C(5), HₐC(5))≈15.8, ³J(H_{b}-C(5), Hₐ-C(6))=9.9, ³J(H_{b}-C(5), H_{b}-C(6))=6.1, 1H, H_{b}C(5)).
^{**13**}**C-RMN** (100 MHz, CDCl₃, 298 K): 211.43 (C(3¹)), 173.13 (C(7)), 172.17 (C(1)), 78.50 (C(4)), 70.63 (C(3²)), 53.48 (C(4^{1'})), 52.07 (C(1¹)), 51.89 (C(7¹)), 50.19 (C(4¹)), 47.24 (C(3)), 31.97 (C(2)), 27.14 (C(6)), 25.66 (C(5))
**MS** (ESI(+)) : [M+Na]⁺ = 354.2
**HR-MS:** 354.1276990 [M+Na]⁺ ; calc. 354.1271711)

### Example 3

### 3-(2-Azido-acetyl)-4-azidomethyl-4-methoxy-heptanedioic-acid dimethyl ester (5):

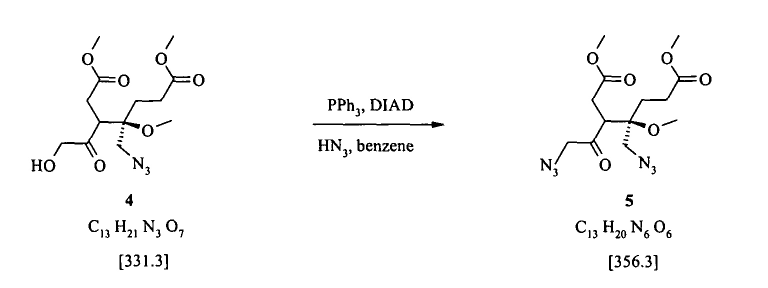

To a solution of 2.329 g (8.8 mmol) of triphenylphosphin (PPh₃) in 95 ml benzene cooled to 10 °C was added drop wise with a syringe 1.795 g (8.8 mmol) of diisopropyl azodicarboxylate (DIAD). After 5 min a solution of 2.675 g (8 mmol) of 4-Azidomethyl-3-(2-hydroxy-acetyl)-4-methoxy-heptanedioic acid dimethyl ester (**4**) in 40 ml benzene was added slowly. After 10 min, 9 ml (10 mmol) of a HN₃ benzene solution was slowly added via syringe. The resulting yellow solution was stirred 2h at 10°C and turned colourless. The solvent was removed under reduced pressure with NaOH trapping the residual hydrogen azide. The oily residue was purified by flash chromatography (CH₂Cl₂ and CH₂Cl₂ / EtOAc 95 : 5) to provide 2.436 g (85%) of the product as a colourless oil. 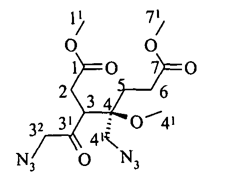 **R**_{**f**} **:** 0.47 (n-hexan /AcOEt 1:1)
**IR (neat)** : 3448w, 2954m, 2841w, 2105vs, 1732s, 1439s, 1414m, 1282s, 1201s, 1179s, 1102m, 1035m, 1010w, 914w, 892w, 873w, 758w, 650w, 556w, 492vw, 440w, 424w, 412w
^{**1**}**H-NMR (400 MHz, CDCl**_{**3**}**, 298 K) :** 4.24 and 4.17 (2xd AB system, ²J= 18.3, 1H each, Hₐ resp. H_{b}C(3²)), 3.71 (s, 3H, H₃C(7¹)), 3.67 (s, 3H, H₃C(1¹)), 3.57 et 3.39 (2xd AB system, ²J= 13.2, 1H each, Hₐ resp. H_{b}C(4^{1'})), 3.26 (dd, ³J= 2.5, ³J= 12.2, 1H , HC(3)), 3.25 (s, 3H, OC(4¹)H₃), 3.0 (dd, ³J= 12.2, ³J= 17.3, 1H , HₐC(2)), 2.44 (dd, ³J= 2.5, ³J= 17.3, 1H , H_{b}C(2)), 2.50-2.27 (m, 2H , H₂C(6)), 2.16 (ddd, J= 5.9, J= 9.4, J= 15.4, 1H HₐC(5)), 1.81 (ddd, J= 6.2, J= 9.7, J= 15.4, 1H , HₐC(5))
^{**13**}**C-NMR (100 MHz, CDCl**_{**3**}**, 298 K)** : 206.07 (C(3¹)), 173.09(C(7)), 172.33 (C(1)), 78.89 (C(4)), 60.06 (C(3²)), 53.32 (C(4^{1'})), 52.16 (C(1¹)), 51.95 (C(7¹)), 50.28 (C(4¹)), 48.54 (C(3)), 32.28 (C(2)), 27.15 (C(6)), 25.48 (C(5))
**MS** (ESI(+)) : [M+Na]⁺ = 379.2
**HR-MS:** 379.13371 [M+Na⁺]calc. 379.1336535)

### Example 4

### 3-[4-Methoxycarbonylmethyl-5-(phenylacetylamino-methyl)-1H-pyrrol-3-yl]-propionic acid methyl ester (7) :

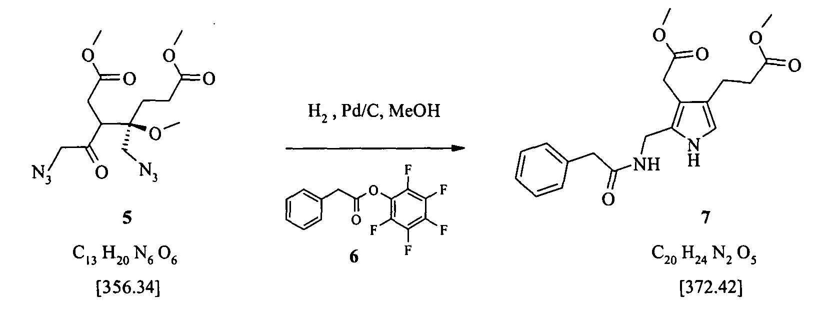

A suspension of 207 mg of Pd/C in 10 ml MeOH was pre hydrogenated at R.T for 15 min, with exclusion of light. A solution of 4.045 g (13.38 mmol) of phenyl-acetic acid pentafluorophenyl ester (**6**) in 20 ml MeOH was added, followed by a solution of 2.385 g (6.69 g) of 3-(2-Azido-acetyl)-4-azidomethyl-4-methoxy- heptanedioic acid dimethyl ester (**5**) in 30 ml MeOH. The resulting suspension was stirred under H₂ for 16 h, at R.T. After filtration over a celite pad and concentration to dryness under reduced pressure, the residue was chromatographed (silica gel, n-hexan / EtOAc 35 : 65) to afford 1.811 g (73%) of the desired product (**7**) as a crystalline solid. 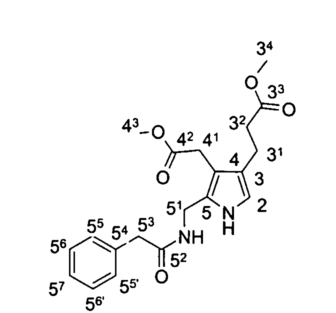 **M.P.** 69-70°C.
**R**_{**f**}**:** n-hexane/AcOEt (35 :65) :0.28 (UV₂₅₄, Ehrlich)
**IR (pastille of KBr)**: 3306s, 3063m, 3030m, 2952m, 1958w, 1754vs, 1648s, 1618s, 1585m, 1537s, 1495m, 1454m, 1436s, 1421m, 1366m, 1331m, 1238m, 1195s, 1162s, 1111m, 1077m, 1056m, 1030m, 1002m, 985m, 938w, 994w, 840w, 797w, 749m, 726m, 697m, 636w, 604m, 527m, 492w, 463w, 426w
^{**1**}**H-NMR (400 MHz, CD**_{**2**}**Cl**_{**2**}**, 298 K) :** 9.00 (s br, 1H, NH-pyrrole), 7.40-7.25 (m, 5H, H aromat.), 6.54 (t br, 1H, NH-amide), 6.44 (d, ³J(2,NH)=2.6, 1H, H-C(2)), 4.28 (d, J(5¹,NH)=5.8, 2H, H₂-C(5¹)), 3.68 (s, 3H, H₃-C(3⁴)), 3.65 (s, 3H, H₃-C(4³)), 3.54 (s, 2H, H₂-C(5³)), 3.44 (s, 2H, H₂-C(4¹)), 2.73 (tripletoïd, ³J(3¹,3²)≈7.7, H₂-C(3¹)), 2.55 (tripletoïd, ³J(3²,3¹)≈7.9, H₂-C(3²))
^{**13**}**C-NMR (100 MHz, CD**_{**2**}**Cl**_{**2**}**, 298 K)**: 173.9 (C(3³)), 173.4 (C(4²)), 172.1 (C(5²)), 135.5 (C(5⁴)), 129.7 (C(5⁵, 5^{5'})), 129.0 (C(5⁶, 5^{6'})), 127.7 (C(5)), 127.4 (C(5⁷)), 121.5 (C(3)), 114.6 (C(2)), 111.9 (C(4)), 52.2 (C(4³)), 51.7 (C(3⁴)), 43.7 (C(5³)), 35.3 (C(5¹)), 35.1 (C(3²)), 30.0 (C(4¹)), 20.8 (C(3¹))
**MS** (ESI(+)) : [M+Na]⁺ = 323.2

| **Anal.** for C₂₀H₂₄N₂O₅ | | | |
|---|---|---|---|
| | C | H | N |
| calc. | 64.50 | 6.50 | 7.52 |
| calc. (+0.16 mol H₂O) | 64.0 | 6.48 | 7.46 |
| found | 63.95 | 6.55 | 7.23 |

### Example 5

### 3-[4-Carboxymethyl-5-(phenylacetylamino-methyl)-1H-pyrrol-3-yl]-propionic acid (8):

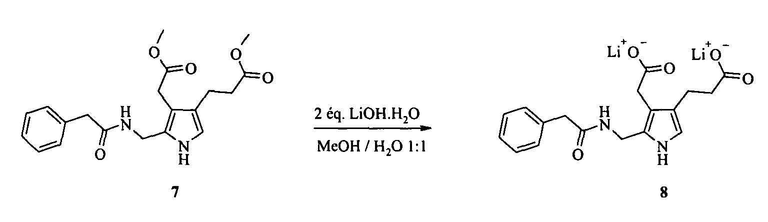

To a solution of 372 mg (1 mmol) of 3-[4-Methoxycarbonylmethyl-5-(phenylacetylamino-methyl)-1H-pyrrol-3-yl]-propionic acid methyl ester (7) in 15 ml of a mixture MeOH / H₂O (1 :1), was added 84 mg (2 mmol) of LiOH.H₂O. This solution was then stirred for 20 h at R.T. and treated by the enzyme with no further purification.
^{**1**}**H-NMR (400 MHz, CD**_{**3**}**OD, 298 K)** : 7.33-7.19 (m, 5H, H aromat.), 6.44 (s, 1H, H-C(2)), 4.28 (s, 2H, H₂-C(5¹)), 3.49 (s, 2H, H₂-C(5³)), 3.35 (s, 2H, H₂-C(4¹)), 2.76 (tripletoïd, ³J(3¹, 3²)≈7.9, H₂-C(3¹)), 2.42 (tripletoïd, ³J(3², 3¹)≈7.9, H₂-C(3²)).
^{**13**}**C-NMR (100 MHz, CD**_{**3**}**OD, 298 K)** : 181.9 (C(3³)), 180.6 (C(4²)),172.5 (C(5²)), 136.2 (C(5⁴)), 129.2 et 128.4 ((C(5⁵) et C(5⁶)), 126.7 (C(5⁷)), 124.3, 123.2 et 116.0 (C(3), C(4) et C(5)), 113.6 (C(2)), 42.8 (C(5³)), 39.0 (C(3²)), 35.6 (C(5¹)), 33.6 (C(4¹)), 22.5 (C(3¹)).
**MS** ESI(-) : [M+H]⁻=343.3

### Example 6

### Phenyl-acetate-4-(2-carboxy-ethyl)-3-carboxymethyl-1H-pyrrol-2-ylmethyl-ammonium (9) :

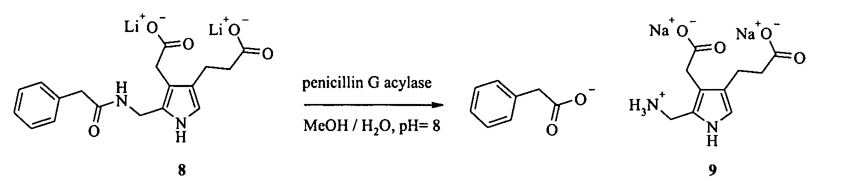

The solution of 3-[4-Carboxymethyl-5-(phenylacetylamino-methyl)-1H-pyrrol-3-yl]-propionic acid di-lithium salt (**8**) in 15 ml of a mixture MeOH / H₂O (1 :1), was diluted with 18 ml H₂O and the pH adjusted to 8 with 0.1 N HCI. A suspension of 420 mg (88 UI) of penicillin G acylase in 25 ml H₂O was added and the resulting mixture was stirred at R.T. for 20 h. The beads of enzyme were then filtered off and the crude residue frozen dried.
^{**1**}**H-NMR (400 MHz, CD**_{**3**}**OD, 298 K)** : 7.33-7.22 (m, 4H, H aromat. PhAcO⁻), 7.19-7.15 (m, 1H, H aromat. PhAcO⁻), 6.53 (s, 1H, H-C(5)), 3.99 (s, 2H, H₂-C(2¹)), 3.49 (s, 2H, H₂C PhAcO⁻), 3.37 (s, 2H, H₂-C(3¹)), 2.75 (tripletoïd, ³J(4¹, 4²)≈7.8, H₂-C(4¹)), 2.40 (tripletoïd, ³J(4², 4¹)≈7.9, H₂-C(4²))
^{**13**}**C-NMR (100 MHz, CD**_{**3**}**OD, 298 K)** : 181.6 (C(4³)), 180.1 (C(3²)), 179.4 (C=O PhAcO⁻), 138.3 (C quaternary aromat. PhAcO⁻), 129.2, 128.1, 125.9 (C aromat. PhAcO⁻), 123.3 (C(4)), 120.8 (C(2)), 118.1 (C(3)), 115.3 (C(5)), 45.4 (CH₂ PhAcO⁻), 39.1(C(4²)), 34.8 (C(2¹)), 33.8 (C(3¹)), 22.3 (C(4¹))
**MS** ESI(-) : [M]⁻=225.2

## Claims

1. Process for the preparation of porphobilinogen (PBG),
**characterized by**
a) reacting lithium hexamethyldisilazane according to following reaction scheme with a 5-hydroxy-4-oxo-pentanoic acid methyl ester of formula 1 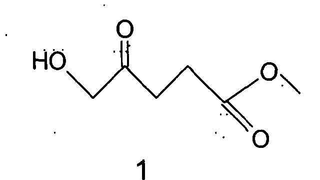 in an inert solvent under cooling at lower temperatures, at temperatures between 0° to -100°C, in the presence of trimethylsilyl chloride obtaining 4,5-bis-trimethylsilanyloxy-pent-3-enoic acid methyl ester of formula 2 in cis- or trans form 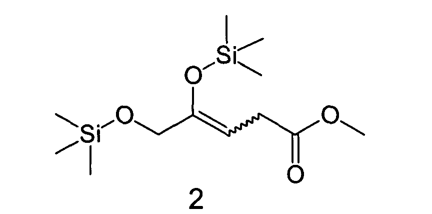
b) The obtained 4,5-bis-trimethylsilanyloxy-pent-3-enoic acid methylester of above formula 2 is reacted with 5-azido-4,4-dimethoxy-pentanoic acid methyl ester of formula 3 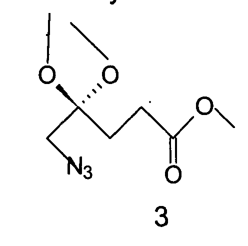 in an inert solvent, in the presence of an acid catalyst and cooling at low temperature of from 0° to -100°C, obtaining 4-azidomethyl-3-(2-hydroxy-acetyl)-4-methoxy-heptanedioic acid dimethyl ester of formula 4 in racemic and stereoisomeric form, e.g. 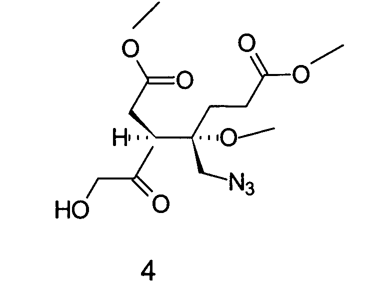
c) 4-azidomethyl-3-(2-hydroxyacetyl)-4-methoxy-heptanedioic acid methyl ester of formula 4 in the presence of triphenylphosphin and dialkylazodicarboxylate and the like in an inert solvent is reacted with HN3 or diphenylphosphorylazide and the like in an inert solvent under cooling obtaining 3-(2-azido-acetyl)-4-azidomethyl-4-methoxy-heptanedioic acid methyl ester of formula 5 in racemic and stereoisomeric form, e.g. 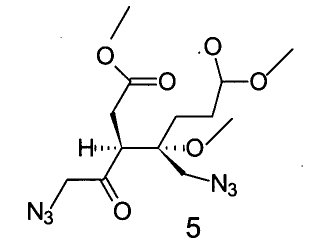
d) The obtained 3-(2-azido-acetyl)-4-azidomethyl-4-methoxy-heptanedioic acid dimethylester of formula 5 is reacted with a pre hydrogenated solution of phenylacetic acid pentafluorophenyl ester of formula 6 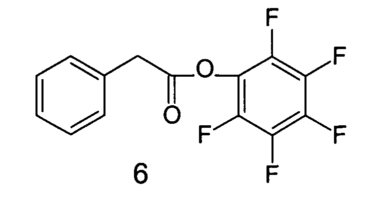 in an inert solvent to afford 3-[4-methoxycarbonylmethyl-5-(protected amino-methyl)-1H-pyrrol-3-yl]-propionic acid methyl ester of formula 7 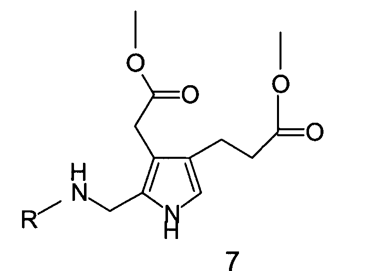 wherein R is a protective group.
e) The obtained 3-[4-methoxycarbonylmethyl-5-(phenylacetylaminomethyl)-1H-pyrrol-3-yl]-propionic acid dimethyl ester of formula 7 is converted with an aqueous/alcoholic alkaline solution into the di-alkali metal salt of formula 8 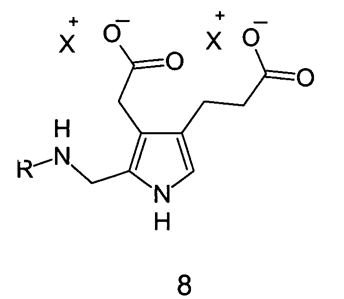
wherein R is a protective group,
and thereafter is hydrolyzed under mild enzymatic conditions or directly by saponification and enzymatic deprotection of a compound of formula 7 to afford porphobilinogen of formula 9 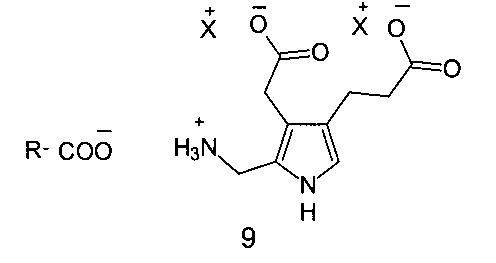 wherein R is a protective group as carboxylate 4-(2-carboxy-ethyl)-3-carboxymethyl-1H-pyrrol-2-yl-methyl-ammonium, and further, if desired, converting the obtained salt into the free amino compound by conventional means, if desired, converting an obtained free amino compound of formula 9 to another corresponding pharmaceutical salt by conventional means.

2. Process according to step a) of claim 1, characterized, that lithium hexamethyldisilazane is reacted with 5-hydroxy-4-oxo-pentanoic acid methyl ester of formula 2 in an inert solvent, e.g. methanol, ethanol, propanol, dichloromethane, dichloroethane, especially in tetrahydrofuran under cooling at lower temperatures, at temperatures between 0° and -100°C, especially at temperatures between -70° and -80°C in the presence of trimethylsilylchloride and methyl jodide.

3. Process according to step a) of claim 1 and claim 2, characterized, that the reaction is carried out in tetrahydrofuran at a temperature of -78°C.

4. Process according to step b) of claim 1, characterized, that 4,5-bis-trimethylsilanyloxy-pent-3-enoic acid methylester of formula 2 is reacted with 5-azido-4,4-dimethoxy-pentanoic acid methyl ester of formula 3 in an inert solvent, e.g. an alcohol, THF, dichloroethane and mixtures thereof, especially in dichloromethane in the presence of titanium chloride under cooling at low temperatures of from 0° to -100°C, especially at temperatures between -70° and -80°C.

5. Process according to step b) of claim 1 and claim 4, characterized, that the reaction is carried out in dichloromethane at a temperature of -78° C.

6. Process according to step c) of claim 1, characterized, that 4-azidomethyl-3-(2-hydroxyacetyl)-4-methoxy-heptanedioic acid methyl ester of formula 4 is reacted in the presence of triphenylphosphin and dialkylazodicarboxylate, especially diisopropylazodicarboxylate in an inert solvent, e.g. methanol, ethanol, propanol, tetrahydrofuran, especially in benzene with HN3 in an inert solvent, especially in benzene under cooling at temperatures of from -20° to +20°C, especially at temperatures of from +5° to +15°C.

7. Process according to step c) of claim 1 and claim 6, characterized, that the reaction is carried out in benzene at a temperature of 10°C.

8. Process according to step d) of claim 1, characterized, that 3-(2-azido-acetyl)-4-azidomethyl-4-methoxy-heptanedioic acid dimethylester of formula 5 is reacted with a pre hydrogenated solution of phenylacetic acid pentafluorophenyl ester of formula 6 in an inert solvent, e.g. methanol, ethanol, propanol, THF or benzene, dichloromethane or dichloroethane and mixtures thereof

9. Process according to step d) of claim 1 and claim 8, characterized, that the reaction is carried out in methanol.

10. Process according to step e) of claim 1, characterized, that 3-[4-methoxycarbonylmethyl-5-(protected aminomethyl)-1H-pyrrol-3-yl]-propionic acid methyl ester of formula 7 is hydrolyzed in aqueous/alcoholic solution, especially in aqueous/methanolic alkaline solution obtaining an alkali metal salt of formula 8 and thereafter treated with an enzyme, especially penicillin G acylase or by treating a compound of formula 7 by enzymatic saponification (hydrolysation) obtaining porphobilinogen which is isolated as carboxylate 4-(2-carboxy-ethyl)-3-carboxymethyl-1H-pyrrol-2-yl-methyl-ammonium of formula 9.

11. Process according to step e) of claim 1 and claim 10,
characterized, that a compound of formula 7, wherein R is a protective group is phenylacetyl, is hydrolyzed in aqueous/methanolic solution in the presence of LiOH.H2O obtaining an di-lithium salt of formula 8a

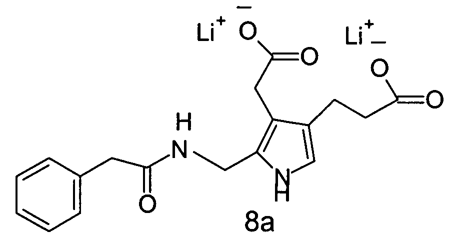

12. 4,5-bis-trimethylsilanyloxy-pent-3-enoic acid methyl ester of formula 2 in cis- or trans form

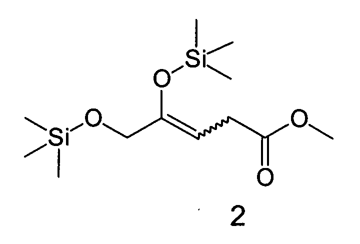

13. Process for the production of 4,5-bis-trimethylsilanyloxy-pent-3-enoic acid methyl ester of formula 2 in cis- or trans form 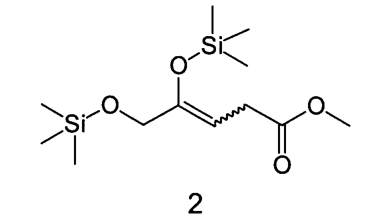 characterized, reacting lithium hexamethyldisilazane with a 5-hydroxy-4-oxo-pentanoic acid methyl ester of formula 1 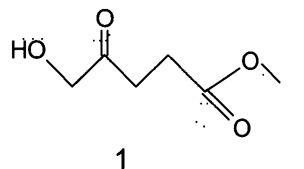 in an inert solvent under cooling at lower temperatures, at temperatures between 0° to -100°C, in the presence of trimethylsilyl chloride and methyljodide.

14. 4-Azidomethyl-3-(2-hydroxy-acetyl)-4-methoxy-heptanedioic acid methyl ester of formula 4 in racemic and stereoisomeric form, e.g.

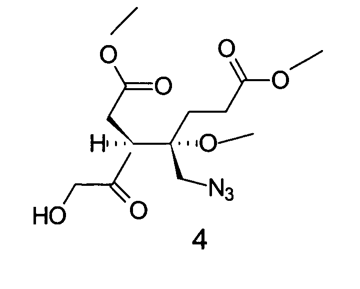

15. Process for the production of 4-azidomethyl-3-(2-hydroxy-acetyl)-4-methoxy-heptanedioic acid dimethyl ester of formula 4 in racemic and stereoisomeric form, e.g. 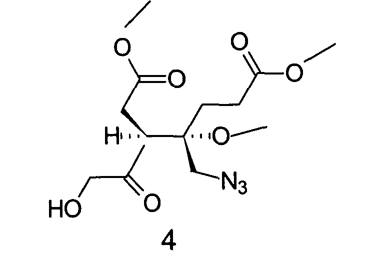 characterized, that 4,5-bis-trimethylsilanyloxy-pent-3-enoic acid methylester of above formula 2 is reacted with 5-azido-4,4-dimethoxy-pentanoic acid methyl ester of formula 3 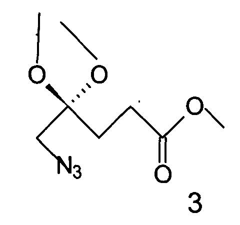 in an inert solvent, in the presence of an acid catalyst, e.g. titaniumchloride and cooling at low temperature of from 0° to -100°C.

16. 3-(2- Azido-acetyl)-4-azidomethyl-4-methoxy-heptanedioic acid methyl ester of formula 5 in racemic and stereoisomeric form, e.g.

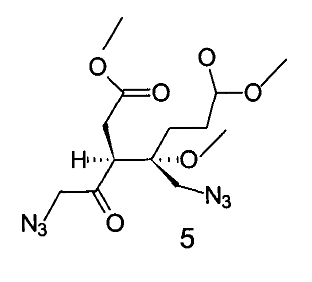

17. Process for the production of 3-(2-azido-acetyl)-4-azidomethyl-4-methoxy-heptanedioic acid methyl ester of formula 5 in racemic and stereoisomeric form, e.g. 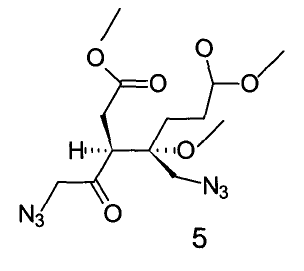 characterized, that 4-azidomethyl-3-(2-hydroxyacetyl)-4-methoxyheptanedioic acid methyl ester of formula 4 is reacted in the presence of triphenylphosphin and dialkylazodicarboxylate and the like in an inert solvent with HN3 or diphenylphosphorylazide and the like in an inert solvent under cooling.

18. 3-[4-Methoxycarbonylmethyl-5-(protected amino-methyl)-1H-pyrrol-3-yl]-propionic acid methyl ester of formula 7 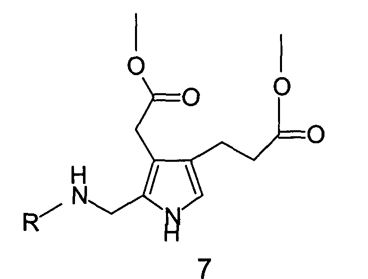 wherein R is a protective group.

19. Process for the production of 3-[4-Methoxycarbonylmethyl-5-(protected amino-methyl)-1H-pyrrol-3-yl]-propionic acid methyl ester of formula 7 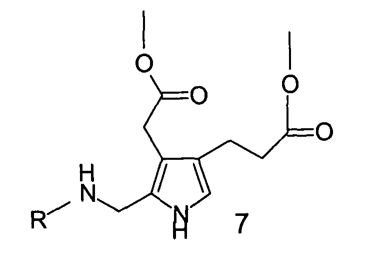 wherein R is a protective group,
characterized, that 3-(2-azido-acetyl)-4-azidomethyl-4methoxyheptanedioic acid dimethylester of formula 5 is reacted with a pre hydrogenated solution of phenylacetic acid pentafluorophenyl ester of formula 6 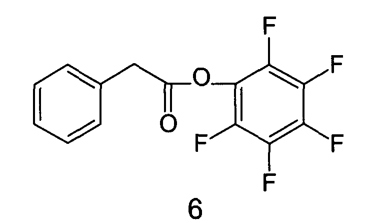 in an inert solvent.

20. 3-[4-Carboxymethyl-5-(protected amino-methyl)-1H-pyrrol-3-yl]-propionic acid di-alkali metal salt of formula 8

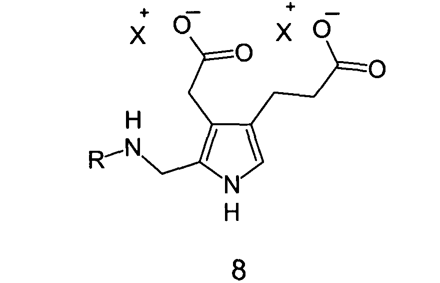

21. 3-[4-Carboxymethyl-5-(phenylacetylamino-methyl)-1H-pyrrol-3-yl]-propionic acid di-lithium salt of formula 8a

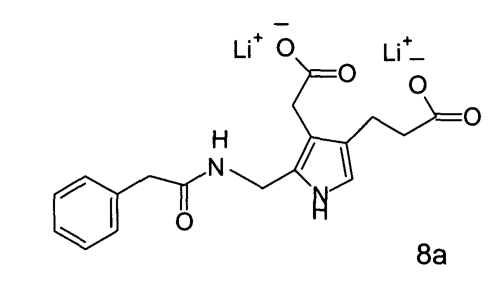

22. Process for the production of 3-[4-Carboxymethyl-5-(protected amino-methyl)-1H-pyrrol-3-yl]-propionic acid di-alkali metal salt of formula 8 or di-lithium salt of formula 8a, wherein R is a phenylacetyl group, 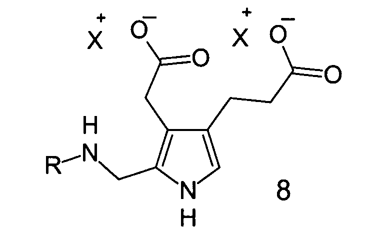 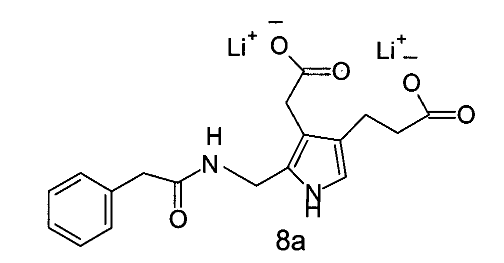 characterized, that 3-[4-methoxycarbonylmethyl-5-(protected aminomethyl)-1H-pyrrol-3-yl]-propionic acid dimethyl ester of formula 7 is reacted with an aqueous/alcoholic alkaline solution, and especially in case of obtaining a compound of formula 8a with an aqueous/alcoholic solution LiOH.H2O for obtaining the di-lithium salt, wherein R as protective group is the phenylacetyl group.

23. Carboxylate 4-(2-carboxy-ethyl)-3-carboxymethyl-1H-pyrrrol-2-ylmethyl-ammonium of formula 9

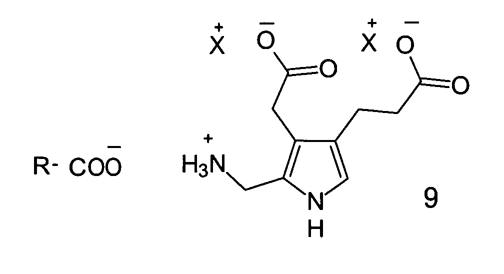

24. Process for the production of carboxylate 4-(2-carboxy-ethyl)-3-carboxymethyl-1H-pyrrrol-2-ylmethyl-ammonium of formula 9 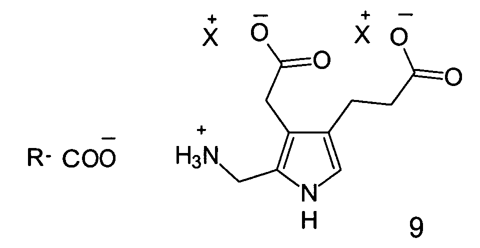 wherein R is a protective group,
characterized, that a compound of formula 8 is hydrolyzed under mild enzymatic conditions or obtained directly by saponification and enzymatic deprotection of a compound of formula 7 to afford porphobilinogen of formula 9.

25. Process for the production of phenylacetate 4-(2-carboxy-ethyl)-3-carboxymethyl-1H-pyrrol-2-yl-methyl-ammonium of formula 9a 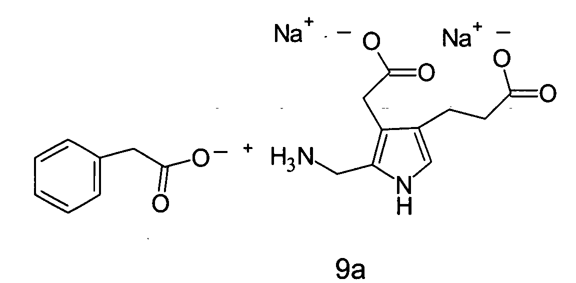 characterized, that a compound of formula 8a is hydrolyzed under mild enzymatic conditions or obtained directly by saponification and enzymatic deprotection of a compound of formula 7 to afford porphobilinogen of formula 9a.

26. Use of a compound of formula 8 or 8a for preparing a protected compound of formula 9 or 9a as ready to use porphobilinogen precursor.

27. Use of a compound of formula 9 or 9a to obtain porphobilinogen in free form for medical treatment.
